# EUROPÄISCHE PATENTSCHRIFT

(11) **EP 4 255 520 B1**
(45) Veröffentlichungstag und Bekanntmachung des Hinweises auf die Patenterteilung: **09.10.2024**
(21) Anmeldenummer: 21831256.9
(22) Anmeldetag: 03.12.2021
(51) Int. Cl.: A61M 1/16

(54) **MEMBRANGASAUSTAUSCHER**
MEMBRANE GAS-EXCHANGER
ECHANGEUR DE GAZ À MEMBRANE

(30) Priorität: 07.12.2020 DE 102020132472
(43) Veröffentlichungstag der Anmeldung: 11.10.2023
(73) Patentinhaber: Fresenius Medical Care Deutschland GmbH, 61352 Bad Homburg (DE)
(72) Erfinder: KLEWINGHAUS, Jürgen, 61440 Oberursel (DE)
(74) Vertreter: Herrmann, Uwe
(86) Internationale Anmeldenummer: PCT/EP2021/084166
(87) Internationale Veröffentlichungsnummer: WO 2022/122584

(56) Entgegenhaltungen:
- DE-A1- 2 803 344
- DE-C2- 2 746 542
- JP-A- H07 255 839

## Beschreibung

Die vorliegende Erfindung betrifft einen Membrangasaustauscher mit einem Gehäuse, in dem eine erste Kammer und eine zweite Kammer sowie eine Membran angeordnet sind, wobei die Membran gasdurchlässig und flüssigkeitsundurchlässig ist und die erste Kammer und die zweite Kammer voneinander trennt, wobei die erste Kammer die Blutseite und die zweite Kammer die Gasseite des Membrangasaustauschers bildet, und wobei die erste Kammer einen Bluteinlass und einen Blutauslass aufweist und wobei die zweite Kammer einen Gaseinlass und einen Gasauslass aufweist, und wobei der Bluteinlass, der Blutauslass, der Gaseinlass und der Gasauslass an dem Gehäuse angeordnet sind.

Derartige Membrangasaustauscher sind aus dem Stand der Technik bekannt. Aus dem Dokument JP-H07-255839A ist ein Membrangasaustauscher mit Dialysatorgehäuse bekannt.

Sie kommen beispielweise in Geräten zur extrakorporalen Dekarboxylierung von Blut (ECCO2R) oder auch bei Geräten zur extrakorporalen Membranoxygenierung von Blut (ECMO) zum Einsatz. Diese Geräte werden eingesetzt, wenn eine künstliche Beatmung erforderlich ist bzw. wenn eine respiratorische Insuffizienz vorliegt, so dass über die Lunge kein hinreichender Gasaustausch (Sauerstoffversorgung des Blutes und CO₂-Abfuhr aus dem Blut) mit dem Blut möglich ist.

Bei der extrakorporalen Decarboxylierung und der extrakorporalen Membranoxygenierung wird das Blut durch einen extrakorporalen Kreislauf und durch die Blutseite des darin befindlichen Membrangasaustauschers geführt und dabei Kohlendioxid aus dem Blut durch den Gasauslass des Membrangasaustauschers abgeführt und Sauerstoff durch den Gaseinlass des Membrangasaustauschers zugeführt. Das Gerät pumpt mittels einer im extrakorporalen Kreislauf befindlichen Blutpumpe Blut kontinuierlich durch den Membrangasaustauscher, der den Gasaustausch in der Lunge ersetzt. Das so aufbereitete Blut wird dann aus dem extrakorporalen Kreislauf zum Patienten zurückgeführt. Mit sinkendem Blutfluss wird der Anteil der Oxygenierung kleiner und der Anteil der Decarboxylierung größer. Die Sauerstoffzufuhr kann bei niedrigem Blutfluss auch direkt erfolgen, über eine Maskenbeatmung, eine Sauerstoff-Nasenbrille oder einen Tubus in der Luftröhre.

Ein Nachteil bekannter Membrangasaustauscher besteht in den vergleichsweise hohen Herstellkosten, die auf die hohen Kosten für das Membranmaterial sowie darauf zurückzuführen sind, dass die Membrangasaustauscher nur in relativ geringen Stückzahlen gefertigt werden und somit nur ein geringer Grad der Automatisierung vorhanden ist.

Der vorliegenden Erfindung liegt die Aufgabe zugrunde, einen Membrangasaustauscher der eingangs genannten Art dahingehend weiterzubilden, dass dieser kostengünstig herstellbar ist.

Diese Aufgabe wird durch einen Membrangasaustauscher mit den Merkmalen des Anspruchs 1 gelöst. Danach ist vorgesehen, dass es sich bei dem Gehäuse des Membrangasaustauschers um das Gehäuse eines Dialysators handelt und dass ein erster Adapter vorgesehen ist, der einen Einlass und wenigstens zwei Auslässe aufweist, wobei der Einlass mit dem Gasauslass des Gehäuses verbunden ist.

Erfindungsgemäß ist somit vorgesehen, dass das Gehäuse eines Dialysators für einen Membrangasaustauscher verwendet wird. Somit kann auf die Produktionsmittel der Diaysatorfertigung hinsichtlich der Gehäuse und ggf. auch hinsichtlich der Membranen zurückgegriffen werden, die gegenüber herkömmlichen Dialysatormembranen z.B. durch eine Beschichtung flüssigkeitsdicht gemacht sind.

Vorzugsweise werden unveränderte Dialysatorgehäuse für den Membrangasaustauscher gemäß der Erfindung verwendet. Diese Gehäuse bzw. deren Ein- bzw. Auslässe für Dialysat sind für die Installation um Umfeld der Dialyse standardisiert, passen aber nicht zu den Anforderungen an Membrangasaustauscher. Zur Anpassung auf die Anforderungen an Membrangasaustauscher ist ein erster Adapter vorgesehen, der einen Einlass und wenigstens zwei Auslässe aufweist, wobei der Einlass mit dem Gasauslass des Gehäuses verbunden ist.

Der Einlass des ersten Adapters ist somit mit dem Gasauslass des Membrangasaustauschers verbunden. Der Gasauslass ist der Dialysatauslass oder -einlass eines für die Dialyse verwendeten Dialysators.

Wird ein Membrangasaustauscher mit einem Standardgehäuse eines Dialysators verwendet, ist es wichtig, dass der Gasauslass keinesfalls verschlossen wird. Wird der Gasauslass verschlossen, kann dies zu einer Herabsetzung der Eliminiationsleistung von CO₂ führen und aufgrund des Druckanstiegs in der zweiten Kammer zu einem ungewollten Gasübertritt auf die Blutseite, d.h. in die erste Kammer des Membrangasaustauschers, was den Patienten erheblich gefährden kann, beispielsweise durch Gasblasen im Blutstrom.

Vor diesem Hintergrund ist vorgesehen, dass der erste Adapter zumindest zwei Auslässe aufweist, so dass die Wahrscheinlichkeit für einen Verschluss des Gasauslasses des Membrangasaustauschers erheblich verringert ist. Durch den Einsatz des ersten Adapters wird ein übliches Dialysatorgehäuse bei der Verwendung als Gehäuse eines Membrangasaustauschers sicherer gestaltet. Wenigstgens ein Auslass kann als nicht-plane Fläche mit Ausbuchtungen zur Erhöhung der Sicherheit ausgebildet sein. So kann ein Verschließen der Öffnung durch Andrücken einer planen Fläche verhindert werden.

Durch die Verwendung eines üblichen Dialysatorgehäuses als Gehäuse für einen Membrangasaustauscher kann hinsichtlich der Produktionskosten von den erheblichen Stückzahlen von Dialysatoren profitiert werden, da die Fertigungseinrichtungen zur Fertigung der Gehäuse nicht geändert werden müssen.

Der erste Adapter enthält die Funktionalität, dass der Gasauslass nicht verschlossen werden kann.

Vorzugsweise ist der erste Adapter lösbar mit dem Gasauslass verbunden. Er kann mit diesem beispielsweise verrastet oder mit einem Schnappverschluss verbunden sein.

In einer bevorzugten Ausführungsform ist der erste Adapter auf den Gasauslass aufgesteckt.

Wie bei einem herkömmlichen Dialysator kann vorgesehen sein, dass die Membran als Hohlfaserbündel vorliegt, dessen Enden in Vergussmassen aufgenommen sind, wie dies aus herkömmlichen Dialysatoren bekannt ist. Dabei ist vorzugsweise vorgesehen, dass der Innenraum der Hohlfasern die erste Kammer, d.h. die Blutkammer bildet und der das Hohlfaserbündel umgebende Raum die zweite Kammer, d.h. die Gasseite bildet.

Die Membran kann einen Grundkörper und eine darauf befindliche flüssigkeitsundurchlässige Beschichtung aufweisen, so dass verhindert wird, das flüssige Bestandteile des Blutes in die zweite Kammer gelangen.

Vorzugsweise weist der Membrangasaustauscher einen Gaseinlass auf, der mit einem zweiten Adapter verbunden ist. Der Gaseinlass dient beispielsweise zur Zufuhr von reinem Sauerstoff, Luft oder einer Gasmischung als Spülgas..

Auch der zweite Adapter ist vorzugsweise lösbar mit dem Gaseinlass verbunden und vorzugsweise auf diesen aufgesteckt. Denkbar ist es weiterhin, dass der zweite Adapter anders ausgeführt ist als der erste Adapter. Mittels des ersten Adapters, über den Gas aus der zweiten Kammer abgeführt wird, muss sichergestellt werden, dass dieser Gasabfluss nicht verschlossen wird. Diese Anforderung besteht für den zweiten Adapter, mittels dessen Gas in die zweite Kammer eingeführt wird, nicht.

Der zweite Adapter kann einen Einlass und einen Auslass aufweisen, wobei der Auslass des zweiten Adapters mit dem Gaseinlass verbunden ist.

Vorzugsweise ist vorgesehen, dass im Bereich des Einlasses des zweiten Adapters ein Halteprofil vorhanden ist, mittels dessen ein Schlauch an dem Einlass fixierbar ist. Das Halteprofil ist beispielsweise ein Tannenbaumprofil, ein Sägezahnprofil etc..

Der erste Adapter kann über einen Schnellverschluss zum Ankoppeln an den Gasauslass verfügen, so dass dieser leicht bedienbar auf den Gasauslass eines Dialysators aufgeschnappt werden kann. Entsprechendes kann alternativ oder zusätzlich für den zweiten Adapter gelten.

Denkbar ist es, dass der erste Adapter über einen Gasprobenauslasss verfügt. So kann ein Messgas zur Bestimmung der Gasbestandteile im abströmenden Gas, z.B. zur Bestimmung des CO₂-Partialdrucks gewonnen werden.

Auch ist es denkbar, dass der erste Adapter über einen Ablauf für Kondenswasser verfügt. Somit kann ein Anschluss, z.B. ein Lueranschluss bereitgestellt werden, um eine Auffangmöglichkeit, z.B. einen Leerspülbeutel für Kondenswasser zu schaffen.

Die vorliegende Erfindung betrifft des Weiteren eine Vorrichtung zur extrakorporalen Membranoxygenierung oder zur extrakorporalen Dekarboxylierung oder zur sonstigen Be- und/oder Entgasung von Blut mit einem extrakorporalen Blutkreislauf, wobei der extrakorporale Blutkreislauf mit dem Bluteinlass und Blutauslass eines Membrangasaustauschers gemäß der vorliegenden Erfindung verbunden ist.

Der zweite Adapter des Membrangasaustauschers ist zum Zwecke der Blutoxygenierung vorzugsweise mit einer Sauerstoffquelle verbunden. Er kann alternativ oder zusätzlich mit einer Spülgasquelle oder mit einer sonstigen Gasquelle verbunden sein.

Denkbar ist es weiterhin, dass der erste Adapter des Membrangasaustauschers mit einer CO₂-Abfuhr verbunden ist, so dass eine hinreichende Dekarboxylierung des Blutes möglich ist.

Die vorliegende Erfindung betrifft des Weiteren die Verwendung eines Dialysatorgehäuses für einen Membrangasaustauscher in einem Gerät zur extrakorporalen Membranoxygenierung und/oder zur extrakorporalen Dekarboxylierung oder zur sonstigen Be- oder Entgasung von Blut.

Weitere Einzelheiten und Vorteile der Erfindung werden anhand eines in der Zeichnung dargestellten Ausführungsbeispiels näher erläutert. Es zeigen:
- Figur 1:: eine schematische Ansicht des ersten Adapters,
- Figur 2:: eine schematische Ansicht des zweiten Adapters und
- Figur 3:: eine schematische Ansicht eines Gehäuses eines bekannten Dialysators.

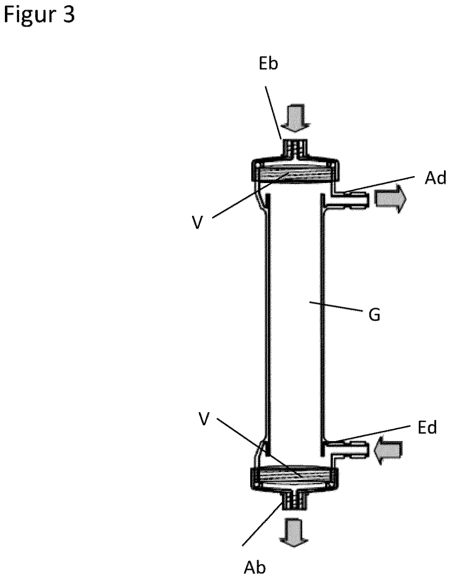

In dem Ausführungsbeispiel wird das Gehäuse G eines herkömmlichen Dialysators verwendet, wie dieses in Figur 3 exemplarisch dargestellt ist. Dieses weist einen Einlass Eb und einen Auslass Ab für Blut auf sowie einen Einlass Ed und einen Auslass Ad für Dialysat. Zwischen den Vergussmassen V erstreckt sich ein nicht dargetelltes Hohlfaserbündel, dessen Innenräume mit der Blutseite und somit auch mit Eb und Ab in Fluidverbindung stehen und dass von einem Raum umgeben ist, der seinerseits mit Ed und Ad in Fluidverbindung steht.

Der Auslass Ad oder der Einlass Ed für Dialysat wird erfindungsgemäß als Gasauslass eines Membrangasaustauschers verwendet, der seinerseits zur Be- und/oder Entgasung von Blut in einem extrakorporalen Kreislauf eines ECMO- oder ECCO2R-Gerätes dient. Wie aus Figur 3 ersichtlich sind Auslass Ad und der Einlass Ed jeweils als von dem Gehäuse G vorspringende Stutzen ausgebildet.

Das Gehäuse G ist vorzugsweise zylindrisch und weist an seinen Stirnseiten den Bluteinlass Eb und den Blutauslass Ab auf und an seiner Mantelfläche den Einlass Ed und Auslass Ad für Dialysat bzw. (im Falle der Verwendung als Membrangasaustauscher) den Einlass und Auslass für Gas auf. Vorzugsweise sind alle Ein- und Auslässe als von dem Gehäuse G hervorragende Stutzen ausgeführt, wie dies aus Figur 3 hervorgeht.

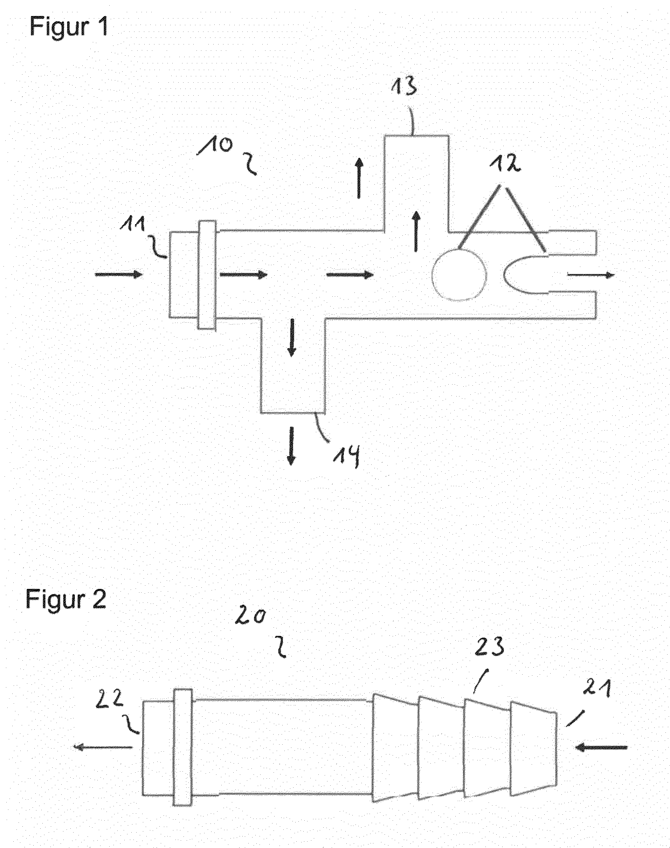

Auf den Gasauslassstutzen Ad ist der erste Adapter 10 gemäß Figur 1 aufgesteckt und dort mittels eines Schnappverschlusses gesichert.

Der erste Adapter 10 weist einen Einlass 11 für das aus dem Gehäuse (nicht dargestellt) ausströmendes Gas sowie mehrere Gasauslässe 12 auf, durch die das Gas den ersten Adapter 10 verlässt. Wie aus Figur 1 weiter ersichtlich, weist der erste Adapter 10 einen Gasprobenauslass 13 sowie darüber hinaus einen Auslass 14 zum Abführen von Kondenswasser in der zweiten Kammer auf. Durch die mehreren Gasauslässe 12 ist die Wahrscheinlichkeit für einen Verschluss des Membrangasaustauschers auf der Gasauslassseite und somit die Gefährdung des Patienten verringert.

Aus Figur 1 geht weiter hervor, dass der rechts dargestellte Gasauslass 12 nicht als plane, z.B. kreisrunde Fläche ausgebildet ist, sondern sich an die plane Stirnseite in der Mantelfläche erstreckende Ausnehmungen aufweist, wodurch das Verschließen dieses Auslasses durch eine plane Fläche verhindert werden kann.

Mit dem Gaseinlassstutzen Ed des Gehäuses G ist der zweite Adapter 20 gemäß Figur 2 verbunden, wobei dieser vorzugsweise ebenfalls durch einen Schnappverschluss an dem Gaseinlassstutzen Ed gesichert ist. Der zweite Adapter 20 weist einen Einlass 21 z.B. für ein Spülgas oder für Sauerstoff auf sowie einen Auslass 22, der auf den Gaseinlassstutzen Ed des Membrangasaustauschers aufgesteckt ist. Wie aus Figur 2 ersichtlich, ist der Einlass 21 des zweiten Adapters 20 von einem Tannenbaumprofil 23 umgeben, so das auf vergleichsweise einfache Art und Weise ein Schlauch oder dgl. an dem zweiten Adapter 20 fixiert werden kann.

## Patentansprüche

1. Membrangasaustauscher mit einem Gehäuse (G), in dem eine erste Kammer und eine zweite Kammer sowie eine Membran angeordnet sind, wobei die Membran gasdurchlässig und flüssigkeitsundurchlässig ist und die erste Kammer und die zweite Kammer voneinander trennt, wobei die erste Kammer die Blutseite und die zweite Kammer die Gasseite des Membrangasaustauschers bildet, und wobei die erste Kammer einen Bluteinlass (Eb) und einen Blutauslass (Ab) aufweist und wobei die zweite Kammer einen Gaseinlass (Ed) und einen Gasauslass (Ad) aufweist, und wobei der Bluteinlass (Eb), der Blutauslass (Ab), der Gaseinlass (Ed) und der Gasauslass (Ad) an dem Gehäuse (G) angeordnet sind, und wobei es sich bei dem Gehäuse (G) um das Gehäuse eines Dialysators handelt,
**dadurch gekennzeichnet, dass** ein erster Adapter (10) vorgesehen ist, der einen Einlass (11) und wenigstens zwei Auslässe (12) aufweist, wobei der Einlass (11) mit dem Gasauslass (Ad) des Gehäuses verbunden ist.

2. Membrangasaustauscher nach Anspruch 1, **dadurch gekennzeichnet, dass** der erste Adapter (10) lösbar mit dem Gasauslass (Ad) verbunden ist.

3. Membrangasaustauscher nach Anspruch 1 oder 2, **dadurch gekennzeichnet, dass** der erste Adapter (10) auf den Gasauslass (Ad) aufgesteckt ist.

4. Membrangasaustauscher nach einem der vorhergehenden Ansprüche, **dadurch gekennzeichnet, dass** die Membran als Hohlfaserbündel vorliegt.

5. Membrangasaustauscher nach einem der vorhergehenden Ansprüche, **dadurch gekennzeichnet, dass** die Membran einen Grundkörper und eine darauf befindliche flüssigkeitsundurchlässige Beschichtung aufweist.

6. Membrangasaustauscher nach einem der vorhergehenden Ansprüche, **dadurch gekennzeichnet, dass** der Membrangasaustauscher einen Gaseinlass (Ed) aufweist, der mit einem zweiten Adapter (20) verbunden ist.

7. Membrangasaustauscher nach Anspruch 6, **dadurch gekennzeichnet, dass** der zweite Adapter (20) lösbar mit dem Gaseinlass (Ed) verbunden ist und vorzugsweise auf den Gaseinlass (Ed) aufgesteckt ist.

8. Membrangasaustauscher nach Anspruch 6 oder 7, **dadurch gekennzeichnet, dass** der zweite Adapter (20) anders ausgeführt ist als der erste Adapter (10) und/oder dass der zweite Adapter (20) einen Einlass (21) und einen Auslass (22) aufweist, wobei der Auslass (21) mit dem Gaseinlass (Ed) verbunden ist, wobei vorzugsweise vorgesehen ist, dass im Bereich des Einlasses (21) ein Halteprofil (23) vorhanden ist, mittels dessen ein Schlauch an dem Einlass (21) fixierbar ist.

9. Membrangasaustauscher nach einem der vorhergehenden Ansprüche, **dadurch gekennzeichnet, dass** der erste Adapter (10) und/oder der zweite Adapter (20) über einen Schnellverschluss zum Ankoppeln an den Gasauslass (Ad) bzw. an den Gaseinlass (Ed) verfügt.

10. Membrangasaustauscher nach einem der vorhergehenden Ansprüche, **dadurch gekennzeichnet, dass** der erste Adapter (10) über einen Gasprobenauslass (13) verfügt.

11. Membrangasauschtauscher nach einem der vorhergehenden Ansprüche, **dadurch gekennzeichnet, dass** der erste Adapter (10) über einen Ablauf (14) für Kondenswasser verfügt.

12. Vorrichtung zur extrakorporalen Membranoxygenierung oder zur extrakorporalen Dekarboxylierung oder zur sonstigen Be- und/oder Entgasung von Blut mit einem extrakorporalen Blutkreislauf, der mit dem Bluteinlass (Eb) und Blutauslass (Ab) eines Membrangasaustauschers gemäß einem der Ansprüche 1 bis 11 verbunden ist.

13. Vorrichtung nach Anspruch 12, **dadurch gekennzeichnet, dass** der zweite Adapter (20) des Membrangasaustauschers mit einer Sauerstoffquelle oder mit einer Spülgasquelle verbunden ist.

14. Vorrichtung nach Anspruch 12 oder 13, **dadurch gekennzeichnet, dass** der erste Adapter (10) des Membrangasaustauschers mit einer CO₂-Abfuhr verbunden ist.

## Claims

1. A membrane gas exchanger having a housing (G) in which a first chamber and a second chamber as well as a membrane are arranged, wherein the membrane is gas permeable and liquid impermeable and separates the first chamber and the second chamber from one another, wherein the first chamber forms the blood side and the second chamber forms the gas side of the membrane gas exchanger, and wherein the first chamber has a blood inlet (Eb) and a blood outlet (Ab), and wherein the second chamber has a gas inlet (Ed) and a gas outlet (Ad), and wherein the blood inlet (Eb), the blood outlet (Ab), the gas inlet (Ed), and the gas outlet (Ad)are arranged at the housing (G),
**characterized in that**
a first adapter (10) is provided that has an inlet (11) and at least two outlets (12), with the inlet (11) being connected to the gas outlet (Ad) of the housing.

2. A membrane gas exchanger in accordance with claim 1, **characterized in that** the first adapter (10) is releasably connected to the gas outlet (Ad).

3. A membrane gas exchanger in accordance with claim 1 or claim 2, **characterized in that** the first adapter (10) is plugged onto the gas outlet (Ad).

4. A membrane gas exchanger in accordance with one of the preceding claims, **characterized in that** the membrane is present as a hollow fiber bundle.

5. A membrane gas exchanger in accordance with one of the preceding claims, **characterized in that** the membrane has a base body and a liquid impermeable coating located thereon.

6. A membrane gas exchanger in accordance with one of the preceding claims, **characterized in that** the membrane gas exchanger has a gas inlet (Ed) that is connected to a second adapter (20).

7. A membrane gas exchanger in accordance with claim 6, **characterized in that** the second adapter (20) is releasably connected to the gas inlet (Ed) and is preferably plugged onto the gas inlet (Ed).

8. A membrane gas exchanger in accordance with claim 6 or claim 7, **characterized in that** the second adapter (20) is differently designed than the first adapter (10); and/or **in that** the second adapter (20) has an inlet and an outlet (22), with the outlet (21) being connected to the gas inlet (Ed), and with provision preferably being made that a retaining section (23) is present in the region of the inlet (21) by means of which retaining section a hose can be fixed to the inlet (21).

9. A membrane gas exchanger in accordance with one of the preceding claims, **characterized in that** the first adapter (10) and/or the second adapter (20) has/have a quick-closure connection for coupling to the gas outlet (Ad) or to the gas inlet (Ed).

10. A membrane gas exchanger in accordance with one of the preceding claims, **characterized in that** the first adapter (10) has a gas sample outlet (13).

11. A membrane gas exchanger in accordance with one of the preceding claims, **characterized in that** the first adapter (10) has an outflow (14) for condensed water.

12. A device for extracorporeal membrane oxygenation or for extracorporeal decarboxylation or for another gassing or degassing of blood using an extracorporeal blood circuit that is connected to the blood inlet (Eb) and blood outlet (Ab) of a membrane gas exchanger in accordance with one of the claims 1 to 11.

13. A device in accordance with claim 12, **characterized in that** the second adapter (20) of the membrane gas exchanger is connected to an oxygen source or to a flushing gas source.

14. A device in accordance with claim 12 or claim 13, **characterized in that** the first adapter (10) of the membrane gas exchanger is connected to a CO₂ discharge.

## Revendications

1. Échangeur de gaz à membrane comportant un boîtier (G) dans lequel sont disposées une première chambre et une seconde chambre ainsi qu'une membrane, la membrane étant perméable aux gaz et imperméable aux liquides et séparant la première chambre et la seconde chambre l'une de l'autre, la première chambre formant le côté sang et la seconde chambre le côté gaz de l'échangeur de gaz à membrane, et la première chambre présentant une entrée de sang (Eb) et une sortie de sang (Ab) et la seconde chambre présentant une entrée de gaz (Ed) et une sortie de gaz (Ad), et l'entrée de sang (Eb), la sortie de sang (Ab), l'entrée de gaz (Ed) et la sortie de gaz (Ad) étant disposées sur le boîtier (G), et le boîtier (G) étant le boîtier d'un dialyseur,
**caractérisé en ce que**
un premier adaptateur (10) est prévu, lequel présente une entrée (11) et au moins deux sorties (12), l'entrée (11) étant reliée à la sortie de gaz (Ad) du boîtier.

2. Échangeur de gaz à membrane selon la revendication 1, **caractérisé en ce que** le premier adaptateur (10) est relié de manière libérable à la sortie de gaz (Ad).

3. Échangeur de gaz à membrane selon la revendication 1 ou 2, **caractérisé en ce que** le premier adaptateur (10) est emboîté sur la sortie de gaz (Ad).

4. Échangeur de gaz à membrane selon l'une des revendications précédentes, **caractérisé en ce que** la membrane se présente sous la forme d'un faisceau de fibres creuses.

5. Échangeur de gaz à membrane selon l'une des revendications précédentes, **caractérisé en ce que** la membrane présente un corps de base et un revêtement imperméable aux liquides se trouvant sur celui-ci.

6. Échangeur de gaz à membrane selon l'une des revendications précédentes, **caractérisé en ce que** l'échangeur de gaz à membrane présente une entrée de gaz (Ed) qui est reliée à un second adaptateur (20).

7. Échangeur de gaz à membrane selon la revendication 6, **caractérisé en ce que** le second adaptateur (20) est relié de manière libérable à l'entrée de gaz (Ed) et est de préférence emboîté sur l'entrée de gaz (Ed).

8. Échangeur de gaz à membrane selon la revendication 6 ou 7, **caractérisé en ce que** le second adaptateur (20) est réalisé différemment du premier adaptateur (10) et/ou **en ce que** le second adaptateur (20) présente une entrée (21) et une sortie (22), la sortie (21) étant reliée à l'entrée de gaz (Ed) et un profil de retenue (23) étant de préférence prévu dans la zone de l'entrée (21), au moyen duquel un tuyau peut être fixé à l'entrée (21).

9. Échangeur de gaz à membrane selon l'une des revendications précédentes, **caractérisé en ce que** le premier adaptateur (10) et/ou le second adaptateur (20) dispose d'un dispositif de fermeture rapide destiné à être couplé à la sortie de gaz (Ad) ou à l'entrée de gaz (Ed).

10. Échangeur de gaz à membrane selon l'une des revendications précédentes, **caractérisé en ce que** le premier adaptateur (10) dispose d'une sortie d'échantillon de gaz (13).

11. Échangeur de gaz à membrane selon l'une des revendications précédentes, **caractérisé en ce que** le premier adaptateur (10) dispose d'un dispositif d'écoulement (14) pour eau de condensation.

12. Dispositif d'oxygénation par membrane extracorporelle ou de décarboxylation extracorporelle ou de tout autre type d'apport de gaz dans le sang et/ou de dégazage du sang avec un circuit sanguin extracorporel qui est relié à l'entrée de sang (Eb) et la sortie de sang (Ab) d'un échangeur de gaz à membrane selon l'une des revendications 1 à 11.

13. Dispositif selon la revendication 12, **caractérisé en ce que** le second adaptateur (20) de l'échangeur de gaz à membrane est relié à une source d'oxygène ou à une source de gaz de rinçage.

14. Dispositif selon la revendication 12 ou 13, **caractérisé en ce que** le premier adaptateur (10) de l'échangeur de gaz à membrane est relié à une évacuation de CO₂.
